# EUROPÄISCHE PATENTSCHRIFT

(11) **EP 2 340 777 B1**
(45) Veröffentlichungstag und Bekanntmachung des Hinweises auf die Patenterteilung: **05.11.2014**
(21) Anmeldenummer: 09180946.7
(22) Anmeldetag: 30.12.2009
(51) Int. Cl.: A61B 17/80

(54) **Osteosyntheseplatte zur Versorgung gelenksnaher Frakturen oder Osteotomien**
Osteosynthesis plate for fractures near joints or osteotomies
Plaque d'ostéosynthèse destinée au traitement de fractures proches d'une articulation ou d'ostéotomies

(43) Veröffentlichungstag der Anmeldung: 06.07.2011
(73) Patentinhaber: Medartis AG, 4057 Basel (CH)
(72) Erfinder: Schonhardt, Jürgen, 79618 Rheinfelden (DE); Glanzmann, Thomas, 1110 Morges (CH); Norström, Joanna, 4053 Basel (CH); Geissler, William B., Brandon, Mississippi 39042 (US)
(74) Vertreter: Hepp Wenger Ryffel AG

(56) Entgegenhaltungen:
- EP-A2- 1 464 295
- WO-A1-03/007832
- WO-A2-98/44849
- WO-A2-2006/031692
- WO-A2-2008/112074
- DE-A1- 10 125 092
- GB-A- 2 451 187
- US-A1- 2007 276 386

## Beschreibung

Die Erfindung betrifft eine Osteosyntheseplatte gemäss dem Oberbegriff des Anspruchs 1. Mit einer derartigen Osteosyntheseplatte wird eine sichere Versorgung einer Fraktur- oder Osteotomiestelle an einem Knochen mit möglichst geringem Risiko von postoperativen Komplikationen angestrebt.

Osteosyntheseplatten zur Implantation, anatomischen Reposition und inneren Schienung von Knochenfragmenten nach Frakturen oder Osteotomien sind weithin bekannt. Es sind mittlerweile Osteosyntheseplatten für die verschiedensten Knochen- und Frakturtypen entwickelt worden.

Eine besondere Herausforderung bei der Entwicklung neuer Osteosyntheseplatten stellen Frakturen und/oder Osteotomien dar, die in der Nähe eines Gelenks liegen, da dort die Knochendichte reduziert ist und besonders viel Weichgewebe in Form von Muskelsehnen und Bändern in den Knochen einstrahlen. Daher sollte eine Osteosyntheseplatte möglichst wenige dieser Weichteile beim Einsetzen der Osteosyntheseplatte verletzen und die Bewegung des Gelenks sollte durch die eingesetzte Osteosyntheseplatte möglichst wenig beeinträchtigt werden. Dabei sollte die Osteosyntheseplatte jedoch noch genug stabil sein, um die in diesem Bereich auf den Knochen einwirkenden Zug- und Torsionskräfte aufzunehmen.

Im Folgenden wird die Problemstellung beispielhaft anhand des Olecranons erläutert.

Das Olecranon ist das proximale Ende der Ulna (Elle). Es ist verdickt und läuft in einen breiten, schnabelartig ausgezogenen Knochensporn (Tuber olecrani) aus. Die obere Fläche des Knochenspornes ist etwa rautenförmig und dort aufgeraut, wo die Sehne des Triceps (Musculus triceps brachii) in den Knochen einstrahlt. Die Behandlung von Frakturen oder Osteotomien im Bereich des Olecranons haben die Wiederherstellung der Gelenkfläche und der Integrität des Bewegungsapparats zum Ziel. Dies erfordert spezielle Fixationen.

Unverschobene und nicht-zertrümmerte Querfrakturen des Olecranons lassen sich relativ einfach durch Ruhigstellung mit einer Oberarmschiene behandeln. Der häufigste Frakturtyp am Olecranon sind jedoch verschobene, nicht-zertrümmerte Frakturen. Die etablierte Behandlungstechnik für solche Frakturen ist die Zuggurtung. Dabei werden zunächst die zwei Knochenfragmente reponiert und zwei parallele Kirschnerdrähte schräg von proximal nach distal eingesetzt. Danach wird ein Loch quer durch das distale Fragment gebohrt. Ein Cerclage-Draht wird dann in Form einer 8 auf der dorsalen Seite der Ulna um die Kirschnerdrähte und durch das distale Querloch geführt. Durch Bildung von Drahtschlingen auf den Seiten lässt sich durch Verdrehen dieser Schlingen die Fraktur komprimieren. Der Cerclage-Draht wandelt dabei Zugkräfte vom Triceps in Druckkräfte im Frakturspalt um.

Bei der Zuggurtung treten häufig Komplikationen auf. Die häufigste Komplikation ist dabei eine schmerzhafte subkutane Prominenz der Drähte, häufig als Folge einer Wanderung der Kirschnerdrähte nach proximal. Probleme können auch die scharfkantigen Drahtenden des Cerclage-Drahts bereiten, die zu Verletzungen oder Reizungen des Weichgewebes führen können. Durch die auftretenden hohen Zugkräfte auf den Cerclage-Draht kann es zudem im Bereich des Austritts des Drahts aus der Bohrung zu Beschädigungen des Knochens oder Ausreissen des Drahtes kommen. Darüber hinaus bietet die Zuggurtung nicht immer genügend Stabilität um die langfristige korrekte Wiederherstellung des Knochens zu gewährleisten. Ausserdem besteht die Möglichkeit, dass der Knochen beim Setzen der Querbohrung ausbricht.

Die Operationstechnik zum Setzen eines Cerclage-Drahts ist teilweise anspruchsvoll, weil verschiedene Parameter gleichzeitig im Auge behalten werden müssen. So sollte die Parallelität der Kirschnerdrähte gewährleistet bleiben während sich der Cerclage-Draht über der Fraktur bez. Osteotomielinie nicht überkreuzen sollte. Das Generieren einer gleichmässigen Kompressionskraft durch Verdrillen des Cerclage-Drahts ist zudem heikel und die Korrektur einer ungleichmässigen Kompression nur schwer durchführbar.

GB 2 451 187 offenbart eine Vorrichtung zum Fixieren von Knochen. Die Vorrichtung verfügt über ein Plattenelement sowie einem Paar von schmalen Spannelementen. Die Spannelemente werden auf einer Seite einer Fraktur mittels Knochenankern befestigt, wobei sie sich über die Fraktur hinweg erstrecken. Auf der anderen Seite der Fraktur werden die Spannelemente durch das Plattenelement am Knochen fixiert, wobei das Plattenelement über Öffnungen für Knochenanker und über Führungen oder Nuten zum Befestigen der Spannelemente verfügt.

WO 2008112074 A2 offenbart eine Platte gemäß dem Oberbegriff von Anspruch 1.

Die US 7,037,308 beschreibt ein Implantat, welches im Wesentlichen auf dem Prinzip der Zuggurtung basiert. Das beschriebene Implantat besteht aus zwei parallel angeordneten, von proximal nach distal in den Knochen und über die Frakturstelle einführbaren Schenkeln, welche über einen auf der Knochenoberfläche anliegenden, drahtähnlichen Abschnitt des Implantats verbunden sind. Der auf der Knochenoberfläche aufliegende Abschnitt erstreckt sich dabei von einem der Schenkel von proximal nach distal über die Frakturstelle hinweg, beschreibt auf dem distalen Fragment einen Bogen und führt danach von distal nach proximal zurück zum zweiten Schenkel. Eine Ausführungsform sieht vor, dass sich die beiden von proximal nach distal und distal nach proximal führenden Teile vor dem Bogen einmal überkreuzen. Das Implantat wird auf dem distalen Fragment mit einer Schraube fixiert. Das Implantat vereint demnach alle Elemente der Zuggurtung in einem Einzelteil.

Nachteilig bei diesem Implantat ist, dass der auf dem Knochen aufliegende Abschnitt sich auf der dorsalen Seite der Ulna befindet, was den Patienten postoperativ dadurch einschränkt, dass Ellbogen und Unterarm nicht schmerzfrei aufgelegt werden können. Ein weiterer Nachteil ist zudem, dass die Zugkräfte nur durch wenige, in den meisten Fällen bloss eine Knochenschraube aufgenommen werden. Dies führt zu einer hohen Belastung des Knochens im Bereich der Schraube. Im Weiteren kann es durch die Lage des Implantats auf der medialen Seite des Knochens zu Irritationen des umliegenden Weichgewebes kommen. Durch diese Lage wird das Implantat auch durch die Haut fühlbar sein, was bei einigen Patienten zumindest für Unbehagen sorgen kann. Ausserdem dürfte für das Setzen dieses Implantats ein Zielinstrument nötig sein.

Bei zertrümmerten, verschobenen und stabilen Frakturen des Olecranons ist bei Patienten unter 60 eine anatomische Reposition der Knochenfragmente mit anschliessender Fixierung durch eine Osteosyntheseplatte die gängigste Behandlungsmethode.

Die Behandlung instabiler Frakturen erfolgt bevorzugt ebenfalls durch Plattenfixierung. Die eingesetzte Osteosyntheseplatte muss bei diesem Frakturtyp zusätzlich zur Aufnahme von Zugkräften eine erhöhte Stabilität aufweisen, da sie mehrere Fragmente überbrücken muss bei denen zudem die interfragmentäre Abstützung fehlt oder beeinträchtigt ist.

Die US 3,716,050 beschreibt eine solche Osteosyntheseplatte. Die Osteosyntheseplatte ist in Form eines "L" ausgebildet und wird auf die dorsale Kante der Ulna fixiert, wobei der kürzere Arm des "L" auf die proximale Fläche des Olecranon zu liegen kommt.

Auf dem Markt ist eine ganze Reihe von Olecranon Osteosyntheseplatten erhältlich, so beispielsweise von Synthes Inc. unter dem Namen "LCP Olecranon" oder von Smith&Nephew unter dem Namen "Peri-LOC Olecranon locking plate". Diese Osteosyntheseplatten liegen alle auf der dorsalen Seite der Ulna und weisen gebogenen Fortsätze auf, die auf die proximale Fläche des Olecranons zu liegen kommen.

Nachteilig an diesen Osteosyntheseplatten ist, dass die gebogenen Fortsätze in den Einstrahlbereich der Sehne des Triceps eingreifen. Daher wird diese Sehne beim Einsetzen der Osteosyntheseplatte teilweise durchtrennt oder zumindest in eine unphysiologische Lage gezwungen. Ein weiterer Nachteil dieser Osteosyntheseplatten ist ihre Position auf der dorsalen Kante der Ulna. Dies behindert den Patienten postoperativ, da der Ellbogen und der Unterarm nicht schmerzfrei aufgelegt werden können. Die über dem Implantat liegende Haut ist so exponiert, dass Hautnekrosen an dieser Stelle nicht aussergewöhnlich sind. Ausserdem können je nach Dicke der Osteosyntheseplatte deren Umrisse durch die Haut sichtbar sein, was bei einigen Patienten Unbehagen verursachen kann. Je nach Länge des Fortsatzes ist zudem eine Einschränkung der Bewegungsfreiheit des Gelenkes durch die Platte möglich. Die oben genannten Nachteile führen mit einer hohen Wahrscheinlichkeit dazu, dass die Implantate nach einiger Zeit wieder entfernt werden müssen, was mit zusätzlichen Kosten verbunden ist und den Patienten durch den zweiten Eingriff erneut belastet.

Die Aufgabe der Erfindung besteht daher darin, ein Implantat der eingangs genannten Art zu schaffen, dass die Nachteile des Bekannten verhindert und eine stabile Versorgung einer Fraktur oder Osteotomie im gelenknahen Bereich mit möglichst geringem Risiko postoperativer Komplikationen ermöglicht. Diese Aufgabe wird erfindungsgemäss mit einer Osteosyntheseplatte mit den Merkmalen des Anspruchs 1 gelöst.

Die erfindungsgemässe Osteosyntheseplatte umfasst mindestens zwei zueinander beabstandete und durch mindestens zwei Stege miteinander verbundene Schenkel. Die Schenkel weisen je eine Länge auf und erstrecken sich in Längsrichtung von einem epiphysären zu einem diaphysären Ende der Osteosyntheseplatte. Ausserdem verfügen beide Schenkel über je mindestens eine Öffnung zur Aufnahme eines Verankerungsmittels. Grösse und Form des mindestens einen Stegs sowie der mindestens zwei Schenkel sind dabei derart ausgebildet, dass die Schenkel beidseitig an einen Knochenanlegbar sind, insbesondere im Bereich eines Gelenks, bevorzugt am Olecranon.

Als Schenkel im Sinne der Anmeldung werden Teile einer Ostosyntheseplatte verstanden, die derart ausgestaltet sind, dass sie über eine Fraktur- oder Osteotomiestelle anlegbar sind. Als Steg werden diejenigen Teile einer Osteosyntheseplatte verstanden, die Schenkel miteinander verbinden und diese in einem definierten Abstand zueinander halten.

Aufgrund der Ausbildung der Platte mit mindestens zwei seitlichen Schenkeln und mit mindestens zwei Stege ist eine stabile Versorgung einer Fraktur- oder Osteotomiestelle im gelenksnahen Bereich eines Knochens möglich, ohne dass dabei die Fläche des Knochens, auf der Sehnen und/oder Bänder einstrahlen durch die Platte abgedeckt wird. Ausserdem bleiben Flächen, die nur durch wenig Weichteile bedeckt werden, aber im Alltag zum Abstützen verwendet werden, von der Platte ausgespart. Der mindestens eine Steg wird dabei so positioniert, dass er diesen Bereich nicht bedeckt. Besonders beim Olecranon wird das Risiko von Hautnekrosen oder Weichteilentzündungen im Bereich der dorsalen Kante der Ulna wesentlich reduziert, da die in diesem Bereich dünne Haut nicht zusätzlich durch eine darunter liegende Platte beansprucht wird.

Unter Grösse werden die äusseren Dimensionen der Schenkel wie Breite, Länge und Dicke, aber auch das Profil verstanden.

Die Schenkel sind derart beschaffen, dass sie sich in allen Ebenen weit genug biegen lassen, um optimal an die Anatomie des Knochens angepasst werden zu können. So kann die Osteosyntheseplatte bei gleichen Dimensionen an die individuellen anatomischen Gegebenheiten eines Patienten angepasst werden. Vor allem können die Schenkel dadurch derart angepasst werden, dass Einstrahlbereiche von Bändern und/oder Sehnen nicht beeinträchtigt werden.

Bei der erfindungsgemässen Ausführungsform der Osteosyntheseplatte sind die mindestens zwei Stege und die mindestens zwei Schenkel derart ausgebildet, dass die mindestens zwei Stege von lateral nach medial über die dorsale Kante des Knochens anlegbar bez. anpassbar ist und sich die mindestens zwei Schenkel an die seitlichen anatomischen Gegebenheiten des Knochens anpassen lassen bez. anpassbar sind. Dadurch wird die Fläche des Knochens, bei der Sehnen und Bänder einstrahlen, von der Platte nicht abgedeckt. Entsprechend werden die Bänder und Sehnen kaum verletzt oder durch die Platte beeinträchtigt. Ein weiterer wesentlicher Vorteil ist zudem, dass durch die Konfiguration der Osteosyntheseplatte mit lateralen Schenkeln und den relativ dünnen Stegen auf der dorsalen Kante die Weichteile, vor allem die Haut, beim Aufstützen nicht zusätzlich beansprucht werden. Bei rein dorsalen Fixationen kann es post-operativ beim Aufstützen zu Schmerzen oder Komplikationen führen.

Als epiphysäres Ende im Sinne der Anmeldung wird dasjenige Ende der Osteosyntheseplatte verstanden, welches bei bestimmungsgemässer Anwendung in Richtung der Epiphyse des Knochens zeigt, also näher am Gelenk liegt. Dementsprechend liegt das diaphysäre Ende der Osteosyntheseplatte in Richtung der Diaphyse des Knochens und somit weiter weg vom Gelenk.

Die Osteosyntheseplatte wird zur Versorgung von Frakturen oder Osteotomien an Knochen verwendet. Gemäss einer ersten Indikation wird die Osteosyntheseplatte zur Versorgung von einfachen Frakturen oder von Osteotomien verwendet, die in der Nähe eines Gelenks liegen. Bei den Knochen handelt es sich um Knochen des menschlichen Bewegungsapparates. Besonders bevorzugt ist die Osteosyntheseplatte derart ausgebildet, dass sie zur Versorgung von Frakturen oder Osteotomien in gelenksnahen Bereichen eines Röhrenknochens anpassbar ist, wie beispielsweise distaler Tibia, proximaler Radius, proximale Ulna oder distaler Humerus. Besonders bevorzugt wird die erfindungsgemässe Osteosyntheseplatte zur Versorgung von Frakturen oder Osteotomien am Olecranon verwendet. Osteotomien im Bereich des Olecranon werden typischerweise vorgenommen, um bei Frakturen des distalen Humerus einen Zugang durch Zurückklappen des Trizeps zu schaffen. Solche einfache Frakturen und Osteotomien werden gemäss dem Stand der Technik typischerweise durch so genannte Zuggurtungen ("tension bands") versorgt. Bei einer solchen Versorgung ist insbesondere das Aufbringen von Kräften in Längsrichtung relevant. Eine besondere Verwindungssteifigkeit der Osteosyntheseplatte ist bei solchen Indikationen weniger relevant.

Gemäss einer anderen Indikation der erfindungsgemässen Osteosyntheseplatte wird die Osteosyntheseplatte zur Versorgung von zertrümmerten oder verschobenen Frakturen eingesetzt. Alternativ kann eine erfindungsgemässe Osteosyntheseplatte auch bei Frakturen eingesetzt werden, bei denen Knochenbereiche nicht wiederhergestellt werden können und so genannte "Defektzonen" vorliegen. In diesen Fällen ist die erfindungsgemässe Osteosyntheseplatte stabiler ausgebildet, so dass sie auch weitere Kräfte aufnehmen kann.

Die Schenkel sind so ausgebildet, dass sie über die äussere Krümmung der Epiphyse durch Biegen anpassbar sind, also teilweise um die Epiphyse des Knochens herumführbar sind. Insbesondere lässt sich so ein Fixationselement, das durch eine Öffnung, die im Bereich der Epiphyse liegt, in etwa in distaler Richtung in den Knochen einsetzen. Dies ermöglicht eine weitere interfragmentäre Kompression.

Bevorzugt sind die mindestens zwei Schenkel und der mindestens eine Steg derart angeordnet, dass auf einer Seite der Frakturlinie ein Bereich durch die Platte nicht abgedeckt wird. Dieser Bereich sollte sich von der Kante der Epiphyse her minimal 20mm, bevorzugt minimal 30mm in Richtung der Diaphyse des Knochens erstrecken. Eine solche Konfiguration hat den Vorteil, dass der Einstrahlbereich für Bänder und Sehnen im gelenksnahen Knochen nicht von der Platte verdeckt wird.

Die Schenkel der Osteosyntheseplatte können in bereits vorgebogenen Zustand vorliegen und/oder während der Operation durch den Chirurgen von Hand und/oder mit einem Biegewerkzeug gebogen werden.

Die Schenkel liegen vor allem für Anwendungen zur Versorgung von Frakturen bevorzugterweise in Form von länglichen Platten vor. Bevorzugt sind die Kanten und Ecken dieser Platten abgerundet. Eine Ausführungsform der Erfindung sieht vor, dass die Schenkel in der Plattenebene wellenförmig ausgebildet sind. Eine weitere Ausführungsform sieht zudem vor, dass die Schenkel zwischen den Bereichen mit den Öffnungen verjüngt sind. Eine alternative Ausführungsform sieht zudem vor, dass die Schenkel zwischen den Bereichen mit den Öffnungen in Form eines Drahtes oder mehrere nebeneinander liegender Drähte vorliegen. Weiter können die Schenkel in Form eines Bandes oder mehrerer nebeneinander liegenden Bänder ausgestaltet sein.

Der Abstand der Schenkel zueinander kann je nach der Indikation, für die die Osteosyntheseplatte vorgesehen ist, variieren. Er sollte jedoch stets so gross sein, dass die beiden Schenkel seitlich an den Knochen angelegt werden können. Eine besondere Ausführungsform sieht vor, dass sich der Abstand zwischen den Schenkeln über die Länge der Osteosyntheseplatte vergrössert und/oder verkleinert. Damit kann die Platte je nach vorgesehner Indikation so vorgelegt werden, dass die Schenkel über ihre ganze Länge seitlich am Knochen angelegt werden können.

Dabei können die beiden Schenkel in einem bestimmten Radius gekrümmt sein. Eine weitere Ausführungsform der Erfindung sieht zudem vor, dass die Schenkel in der Plattenebene wellenförmig ausgeführt werden, wobei sich der Abstand der Schenkel über die Länge der Osteosyntheseplatte periodisch verändert. In einer ganz besonders bevorzugten Ausführungsform nimmt der Abstand der Schenkel in Längsrichtung zum epiphysären Ende hin zu. Durch diese Anordnung umgehen die mindestens zwei Schenkel die Einstrahlzonen von Sehnen und Bändern des nahen Gelenks und lassen sich auch einfach der seitlichen Anatomie des Knochens anpassen.

Die Schenkel verfügen je über mindestens zwei Öffnungen zur Aufnahme von Fixationselementen. Die Dicke der Schenkel für Anwendungen bei Osteotomien oder einfachen Frakturen ist im Bereich der Öffnungen, das heisst in einem gewissen Radius um die Öffnungen herum, grösser als in den Bereichen zwischen den Öffnungen. Eine andere Ausführungsform, vor allem zur Versorgung von komplizierteren Frakturen, sieht vor, dass die Schenkel über ihre gesamte Fläche gleich dick sind. Der Durchmesser der Öffnugnen ist derart gewählt, dass je nach vorgesehener Indikation genügend starke Fixationselemente eingesetzt werden können.

Geeignete Fixationselemente sind beispielsweise Knochenschrauben, Stifte, Drähte und dergleichen.

Bei einer Osteosyntheseplatte, die für die Versorgung einer Fraktur oder Osteotomie am Olecranon anpassbar ist, beträgt der Durchmesser der Öffnungen zwischen 1.5 mm und 3.5 mm, bevorzugt zwischen 2.5 mm und 3.0 mm.

Bevorzugt ist der Steg weniger dick ausgebildet als die Schenkel im Bereich der Öffnungen, vorzugsweise halb so dick. Besonders bevorzugt ist der Steg weniger dick ausgebildet als die geringste Dicke der Schenkel. Wird die erfindungsgemässe Osteosyntheseplatte derart ausgebildet, dass sie für die Versorgung eines Knochendeffekts am Olecranon anpassbar ist, hat ein möglichst dünner Steg den wesentlichen Vorteil, dass der Patient postoperativ beim Auflegen des Ellenbogens oder des Unterarms kaum durch die eingesetzte Osteosyntheseplatte gestört wird. Ausserdem wird so verhindert, dass die Umrisse der Osteosynthseplatte durch die Haut sichtbar werden. Die Dicke des Stegs sollte bei einer solchen Osteosyntheseplatte zwischen 0.1 mm und 0.7 mm, bevorzugt zwischen 0.2 mm und 0.6 mm betragen. Durch eine solche Anordnung lässt sich der mindestens eine Steg vollständig über die dorsale Kante der Ulna biegen. Damit ist es möglich, die Schenkel beidseitig auf der Ulna zu platzieren, ohne dass diese zu nahe an der dorsalen Kante zu liegen kommen.

Bevorzugt ist der Steg dabei als Platte oder Band ausgebildet. In einer anderen Ausführungsform zur Anwendung z. B. bei Osteotomien kann der Steg aber auch drahtförmig ausgebildet sein. Jedoch sollte der Steg derart ausgebildet sein, dass er sich intraoperativ an die Krümmung der Knochenoberfläche anpassen lässt. Bevorzugterweise lässt sich der mindestens eine Steg von Hand biegen ohne zu knicken.

Die erfindungsgemässe Osteosyntheseplatte kann auch mehr als zwei Stege aufweisen, bevorzugt weist die Osteosyntheseplatte jedoch 2 oder 3 Stege auf. Dabei sind die Stege stets so angeordnet, dass der kürzere Schenkel bevorzugt zu einem ersten Ende der Platte hin, insbesondere dem epiphysären Ende, über mindestens 15mm seiner Länge nicht durch einen Steg mit dem längeren Schenkel verbunden ist. Die Stege sind so angeordnet, dass der kürzere Schenkel zu einem ersten Ende der Platte hin, insbesondere dem epiphysären Ende, über mindestens 60% seiner Länge nicht mit dem längeren Schenkel verbunden ist. Falls beide Schenkel gleich lang sind, ist der mindestens eine Steg derart angeordnet, dass mindestens 15mm oder alternativ mindestens 60% der Länge der Schenkel in Längsrichtung bevorzugt zu einem ersten Ende der Platte hin, insbesondere dem epiphysären Ende, keine Verbindung untereinander haben. So lässt sich die Platte von einer Seite, entweder von epiphysär oder diaphysär über den Bruch schieben, ohne dass der mindestens eine Steg über den Einstrahlbereich der Sehnen und/oder Bänder geschoben wird.

Die Länge des kürzeren Schenkels, die bevorzugt zu einem ersten Ende der Platte hin keine Verbindung zum zweiten bez. längeren Schenkel hat, beträgt bei einer Osteosyntheseplatte für die Versorgung einer Fraktur oder Osteotomie am Olecranon mindestens 15 mm, bevorzugt mindestens 18 mm, besonders bevorzugt mindestens 20 mm. Damit wird verhindert, dass der Steg auf den Einstrahlbereich des Trizeps an die Ulna zu liegen kommt. Die Länge der Schenkel ist dabei aber dennoch ausreichend, um diese bis an das proximale/epiphysäre Ende des Olecranons anzulegen.

Bevorzugt weist der mindestens eine Steg dabei mindestens eine Aussparung, die vorzugsweise mittig im Steg angeordnet ist, auf. Diese mindestens eine Aussparung ermöglicht gegenüber einem Steg ohne Aussparung eine höhere Steifigkeit gegenüber Torsionsbeanspruchungen bei gleicher Querschnittsfläche, ohne die Biegbarkeit zu beeinrächtigen. Ausserdem verringert sich dadurch die Auflagefläche des Implantats auf die Knochenhaut, was für die arterio-venöse Zirkulation von Vorteil ist. Besonders vorteilhaft ist der Steg als Meshstruktur ausgebildet, besteht also aus vielen sich teilweise überkreuzenden Stegen. Durch eine solche Struktur lässt sich der Steg einfacher an die Variabilität der Knochen anpassen.

Der Querschnitt der Schenkel kann eine beliebige Form aufweisen. Bevorzugt weisen die Schenkel einen rechteckigen Querschnitt auf, wobei die Kanten zur Vermeidung von Verletzungen oder Reizungen des Weichgewebes abgerundet sind. Weitere bevorzugte Querschnitte sind runde, halbrunde, trapezförmige oder allgemein polygonale Querschnitte.

Die beiden Schenkel weisen bevorzugt unterschiedliche Längen auf, können aber auch gleich lang sein. Die Länge der Schenkel richtet sich primär nach dem Knochen, für den die Osteosyntheseplatte vorgesehen ist. Vorteilhafterweise kann ein längerer Schenkel je nach Indikation der Platte auftretende Kräfte besser über die Länge des Knochens verteilen oder zur Befestigung weiterer Fragmente eingesetzt werden.

Die Dicke der Schenkel ist vorzugsweise über die gesamte Länge der Schenkel konstant. In einer bevorzugten Ausführungsform kann die Dicke entweder in Richtung des diaphysären und/oder des epiphysären Endes der Osteosyntheseplatte hin abnehmen. Dabei können die Schenkel im Bereich des Stegs die grösste Dicke aufweisen. Besonders bevorzugt sind die Schenkel im Bereich der Öffungen dicker als in den Bereichen zwischen den Öffnungen. Vor allem bei Anwendung bei Osteotomien oder Frakturen mit intrafragmentärer Abstützung kann die Dicke der Schenkel im Vergleich zum Bereich der Öffnungen reduziert sein. Durch die reduzierte Dicke wird das Auftreten von Weichgewebsentzündungen oder gar von Nekrosen minimiert, da im Vergleich zu dickeren Platten weniger Gewebe verdrängt wird.

Bei einer Osteosyntheseplatte, die für die Versorgung einer Fraktur oder einer Osteotomie am Olecranon vorgesehen ist beträgt die Länge eines ersten, längeren Schenkels zwischen 30 mm und 200 mm, besonders bevorzugt zwischen 40mm und 60mm und die Länge des zweiten, kürzeren Schenkels beträgt zwischen 30mm und 50mm, bevorzugt zwischen 30mm und 40mm. Der Abstand zwischen den Schenkeln liegt dabei zwischen 10mm und 40mm, bevorzugt zwischen 10mm und 25mm. Die Dicke der Schenkel beträgt zumindest im Bereich der Öffnungen zwischen 1.0mm und 4.0mm, bevorzugt zwischen 1.5 mm und 2.5 mm.

In einer alternativen Ausführungsform der Erfindung ist der mindestens eine Steg derart angeordnet, dass in Richtung des zweiten Endes der Platte hin, insbesondere dem diaphysären Ende, die Schenkel über mindestens 2.5mm oder alternativ mindestens 10% der Länge des kürzeren Schenkels keine Verbindung untereinander haben.

Bei einer Osteosyntheseplatte, die für die Versorgung einer Fraktur oder einer Osteotomie am Olecranon anpassbar ist, beträgt der Abstand zwischen Steg und diaphysärem Ende des kürzeren Schenkels mindestens 3 mm, bevorzugt mindestens 7 mm, besonders bevorzugt mindestens 10 mm.

Die Platte gemäß der Erfindung weist zwei sich überkreuzende Stege auf, die die beiden Schenkel miteinander verbinden und deren Kreuzungsbereich derart angeordnet ist, dass der Abstand zwischen diesem Bereich und dem einen Ende der Osteosyntheseplatte, insbesondere dem epiphysären Ende, mindestens 15mm der Länge des kürzeren Schenkels entspricht. Dieser Abstand beträgt mindestens 60% der Länge des kürzeren Schenkels.

Die Stege weisen dabei eine Form auf, die im Wesentlichen dem Buchstaben "X" ähnelt. Besonders bevorzugt weisen die beiden Stege dabei eine leichte Krümmung auf. Die Übertragung einer Kompressionskraft auf die Fraktur- oder Osteotomiestelle erfolgt über die gekreuzten und gekrümmten Stege spannungspitzenarm.

Besonders bevorzugt ist mindestens eine der Öffnungen der Schenkel zum Verblocken eines Fixationselements, insbesondere einer Schraube, ausgebildet. Verblockungstechnologien sind auf dem Gebiet der Osteosynthese bekannt. Dadurch lässt sich eine erfindungsgemässe Osteosyntheseplatte winkelstabil auf den Knochen verankern. Ein winkelstabil verankertes Fixationselement kann sich postoperativ z.B. bei physiotherapeutischer Nachbehandlung weniger lösen und zu Weichteilirritationen führen.

Alternativ können die Öffnungen in den beiden Schenkeln zueinander asymmetrisch angeordnet sein. Dies minimiert die Wahrscheinlichkeit des Aufeinandertreffens von Fixationselementen im Innern des Knochens. Ausserdem wird die Wahrscheinlichkeit einer Knochenspaltung oder -absplitterung dadurch ebenfalls minimiert.

Bevorzugt ist mindestens eine Öffnung derart ausgebildet, dass das Einsetzen eines Fixationselements in einem Winkel möglich ist, der von 90° bezogen zur Plattenebene verschieden ist. Bevorzugt ist die Öffnung derart ausgebildet, dass das Einsetzen unter einem Winkel im Bereich von 80° bis 45° zur Plattenebene möglich ist. Die Achse der Öffnung kann dabei selbst in einem Winkel zur Platte stehen. Eine alternative Ausführungsform sieht vor, dass die Platte im Bereich einer solchen Öffnung überhöht ist, um ein Herausragen eines Teils des unter einem Winkel eingesetzten Konchenankers zu verhindern. Eine solche überhöhte Öffnung wird in der EP1861031 beschrieben. Das Einsetzen eines Fixationselements unter einem anderen Winkel als 90° zur Plattenoberfläche ermöglicht eine an die Anatomie und Dichte des Knochens optimal angepasste Verankerung.

Alternativ kann mindestens eine Öffnung als Langloch ausgebildet sein. Dies ermöglicht das Verschieben der Osteosyntheseplatte im Zuge einer anderweitig aufgebrachten Kompressionskraft auf die Fraktur- oder Osteotomiestelle, ohne die Gefahr einer Dislokation der proximalen Fragmente. Besonders bevorzugt ist das Langloch als Kompressionsloch ausgebildet. Dabei wird beim Einschrauben einer Knochenschraube durch die Ausgestaltung des Langlochs eine Kompressionskraft auf die Fraktur- oder Osteotomiestelle ausgeübt.

Jeder Schenkel weist bevorzugt mindestens je eine Öffnung am epiphysären sowie am diaphysären Ende auf. Dadurch lässt sich die Osteosyntheseplatte dreh- und verschiebungsstabil auf den Knochen fixieren. Zwischen diesen Öffnungen können noch weitere Öffnungen angebracht sein.

In einer besonders bevorzugten Ausführungsform der Erfindung ist die Osteosyntheseplatte derart beschaffen, dass mindestens eine Öffnung auf das epiphysäre Fragment des Knochens zu liegen kommt. Eine andere, besonders bevorzugte Ausführungsform der Erfindung sieht vor, dass jeder Schenkel über mehrere, insbesondere 2, 3, 4, 5, 6, oder mehr Öffnungen aufweist. Damit lassen sich beispielsweise zertrümmerte Frakturen besonders gut behandeln, da möglichst viele der Fragmente mit möglichst wenig Fixationselementen fixiert werden können.

Die Ostesyntheseplatte besteht bevorzugt aus einem bioverträglichen Material, insbesondere einem bioverträglichen Metall oder einer bioverträglichen Legierung. Beispielsweise besteht die Osteosyntheseplatte aus Titan, Edelstahl, einer Titanlegierung wie z.B. TAV (Ti6A14V) oder TAN (Ti6A17Nb), einer Zirkoniumlegierung oder einer Magnesiumlegierung. Alternativ kann die Osteosyntheseplatte auch aus einem bioverträglichen, bevorzugt abbaubaren Polymer bestehen.

Die Platte gemäß der Erfindung kann in einem Verfahren zur Versorgung einer Fraktur oder einer Osteotomie im gelenksnahen Bereich eines Knochen, insbesondere des Olecranons benutzt werden. In einem ersten Schritt wird eine wie vorstehend beschriebene Osteosyntheseplatte bereitgestellt. Danach wird zuerst der mindestens eine Steg an die Krümmung eines Knochens im gelenksnahen Bereich, insbesondere der dorsalen Kante der Ulna, durch Biegen angepasst. Ebenso werden die Schenkel durch Biegen an die seitliche Anatomie des Knochens angepasst. Diese Platte wird insbesondere mit Knochenschrauben so am Knochen befestigt, dass die beiden Schenkel seitlich am Knochen anliegen. Besonders vorteilhaft bleibt dabei der Einstrahlbereich der Sehnen und/oder Bänder in den Knochen von der Platte unbedeckt.

Bevorzugt können die epiphysären Enden der Schenkel der Osteosyntheseplatte um das proximale Olecranon herumgeführt und in diesem Bereich ebenfalls mit Fixationselementn befestigt werden.

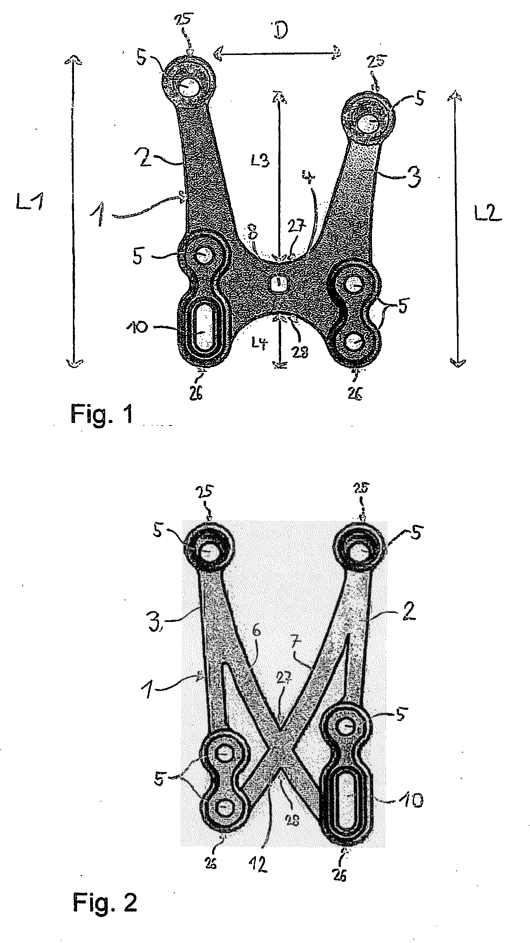

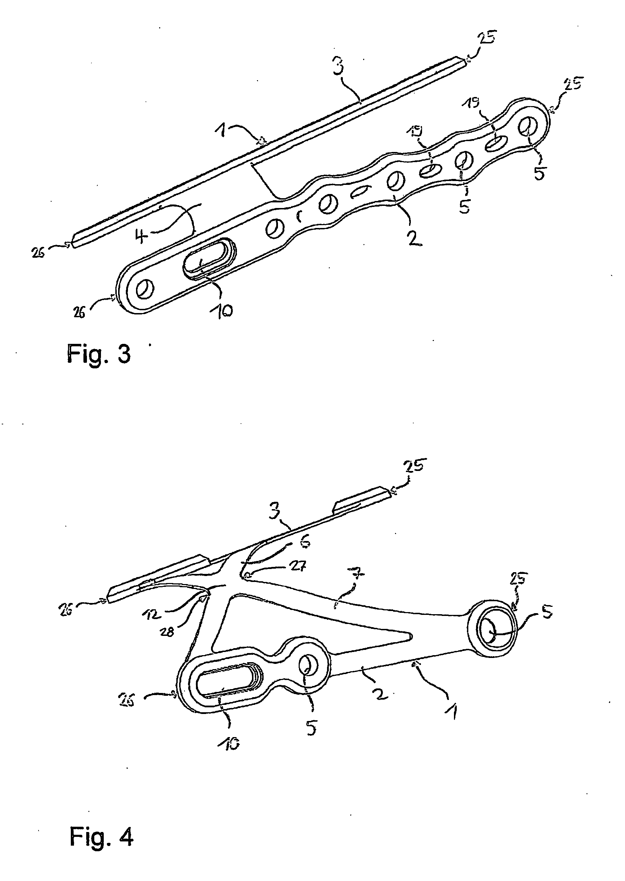

Weitere Vorteile und Einzelmerkmale der Erfindung ergeben sich aus den folgenden Ausführungsbeispielen und den Figuren. Nur die Ausführungsbeispiele der Figuren 2 und 4 fallen unter den Schutzumfang der Ansprüche. Es zeigen:
- Fig. 1:: Eine Ausführungsform einer Osteosyntheseplatte mit einem Steg
- Fig. 2:: Eine erste Ausführungsform der erfindungsgemässen Osteosyntheseplatte mit zwei sich überkreuzenden Stegen
- Fig. 3:: Eine weitere Ausführungsform der Osteosyntheseplatte die einen vorgekrümmten Steg und Aussparungen in den Schenkeln aufweist
- Fig. 4:: Die Osteosyntheseplatte aus Figur 2 in vorgekrümmten Zustand
- Fig. 5:: Noch eine weitere Ausführungsform der Osteosyntheseplatte mit Schenkeln in Form einer Schlangenlinie
- Fig. 6:: Eine perspektivische Darstellung der Osteosyntheseplatte gemäss Fig. 1 zur Versorgung einer Osteotomie am Olecranon
- Fig. 7a bis 7c:: Perspektivische Darstellungen einer weiteren Ausführungsform einer Osteosyntheseplatte zur Versorgung einer Trümmer-Fraktur mit Defektzone am Olecranon.
- Fig. 8:: Eine weitere Ausführungsform der Osteosyntheseplatte mit vorgekrümmten Schenkel
- Fig. 9:: Eine Osteosyntheseplatte mit zwei Stegen
- Fig. 10:: Eine alternative Ausführungsform der Osteosyntheseplatte gemäss Fig. 9
- Fig. 11:: Eine weitere Ausführungsform der Osteosyntheseplatte, bei der die beiden Schenkel über ihr epiphysäres Ende durch einen Steg verbunden sind
- Fig. 12:: Ein Anwendungsbeispiel einer Platte wie nach Fig. 11
- Fig. 13:: Ein Anwendungsbeispiel einer Osteosyntheseplatte mit über der äusseren Krümmung der Epiphyse angelegten Schenkeln.

Figur 1 zeigt eine erste Ausführungsform der Osteosyntheseplatte 1. Das epiphysäre Ende 25 der Platte ist in der Figur oben und das diaphysäre Ende 26 unten dargestellt. Ein längerer Schenkel 2 weist eine Länge L1 und ein kürzerer Schenkel 3 eine Länge L2 auf. Die beiden Schenkel 2, 3 sind durch einen Steg 4 miteinander verbunden. Der Steg 4 ist dabei in Form einer Platte ausgebildet, wobei die epiphysäre Kante 27 sowie die diaphysäre Kante 28 des Stegs 4 eine leichte konkave Krümmung aufweisen. Bei dieser Ausführung weist der Steg 4 eine mittig angeordnete Aussparung 8 auf. Die beiden Schenkel 2, 3 sind nicht gerade ausgebildet, sondern weisen in Längsrichtung vom diaphysären Ende 26 zum epiphysären Ende 25 hin eine leichte Krümmung nach aussen auf. Dadurch nimmt der Abstand D zwischen den Schenkel entsprechend von diaphysär nach epiphysär zu. Je eine Öffnung 5 ist am epiphysären Ende 25 der Schenkel 2, 3 angebracht. Am diaphysären Ende 26 weisen die Schenkel 2, 3 je zwei Öffnungen 5 auf, wobei bei dieser Ausführungsform eine Öffnung 5 des längeren Schenkels 2 als Langloch 10 ausgebildet ist. Die Dicke der Platte ist im Bereich der Öffnungen 5 leicht überhöht und somit grösser als in den Bereichen zwischen den Öffnungen 5.

Typischerweise weist die in Fig. 1 gezeigte Osteosyntheseplatte 1 eine Länge L1 von 43 mm des längeren Schenkels 2 und eine Länge L2 von 38mm des kürzeren Schenkels 3 auf. Im Bereich des Stegs 4 weist die Platte inklusive der Schenkel eine Breite von 27 mm auf.

Die Dicke der Osteosyntheseplatte 1 im Bereich der Schenkel 2, 3 ausserhalb des Bereichs der Öffnungen 5 und im Bereich des Stegs 4 beträgt 0.5 mm. Im Bereich um die Öffnungen 5 beträgt die Plattendicke 1.6 mm.

Die Schenkel 2, 3 sind also im Ausführungsbeispiel gemäss Fig. 1 relativ dünn ausgebildet. Die Schenkel 2, 3 reichen aufgrund ihrer Festigkeit aber ohne weiteres zur Versorgung einer Fraktur oder Osteotomie im gelenksnahen Bereich eines Knochens, insbesondere des Olecranons aus, da sie nur Zugkräfte übertragen müssen.

Es ist ausserdem gemäss einer alternativen Ausführungsform (nicht dargestellt) auch denkbar, die Schenkel 2, 3 drahtartig mit einem runden Querschnitt auszubilden.

Der Steg 4 ist ersichtlich benachbart zum diaphysären Ende der Osteosyntheseplatte 1 angeordnet. Typischerweise kann beispielsweise der Abstand L3 vom epiphysären Ende 25 des kürzeren Schenkels 3 zur epiphysären Kante 27 von Steg 4 etwa 24mm betragen, das heisst, etwa 63% der Länge L2 des kürzeren Schenkels 3. Der Abstand L4 zwischen dem diaphysären Ende 26 des kürzeren Schenkels 3 der Osteosyntheseplatte 1 und der diaphysären Kante 28 des Stegs 4 beträgt hingegen typischerweise nur etwa 7mm.

In Figur 2 ist eine Ausführungsform einer erfindungsgemässen Osteosyntheseplatte 1 zu sehen. Die Platte 1 ist mit zwei sich überkreuzenden Stegen 6, 7 versehen. Die beiden Stege sind beide in einem Radius leicht gekrümmt. Bei dieser Ausführung ist der Kreuzungsbereich 12 dabei derart angeordnet, dass die epiphysäre Kante 27 die durch das Aufeinandertreffen der beiden Stege 6, 7 entsteht, einen Abstand vom epiphysären Ende 25 der Platte einnimmt, der etwa 60% der Länge des kürzeren Schenkels 3 beträgt. Anzahl und Anordnung der Öffnungen 5 sowie des Langlochs 10 entsprechen bei dieser Ausführungsform derjenigen der Ausführungsform in Figur 1.

Figur 3 zeigt eine weitere alternative Ausführungsform einer Osteosyntheseplatte 1. Der Steg 4 der Osteosyntheseplatte 1 ist dabei bereits vorgekrümmt. Die beiden Schenkel 2, 3 sind nicht gerade ausgebildet, sonder bereits leicht den Konturen des seitlichen Knochens angepasst. Die Breite der Schenkel 2, 3 ist im Bereich zwischen den Öffnungen 5 etwas kleiner als im Bereich der Öffnungen 5. In diesen Zwischenbereichen weist diese Ausführungsform der Osteosyntheseplatte 1 Aussparungen 19 auf, die für eine leichtere Biegbarkeit sorgen. Eine der Öffnungen 5 ist dabei wieder als Langloch 10 ausgebildet.

In Figur 4 ist die Osteosyntheseplatte 1 aus der Figur 2 in vorgekrümmten Zustand abgebildet. Dabei sind die beiden sich überkreuzenden Stege 6, 7 entsprechend der Kontur des Knochens gebogen. Auf dieser Figur ist gut zu sehen, dass die Schenkel im Bereich zwischen den Öffnungen 5 sowie die Stege 6, 7 dünner ausgebildet sind als die Schenkel 6, 7 im Bereich um die Öffnungen 5 herum. Da bei einfachen Frakturen vor allem Zugkräfte zu übertragen sind, können die Schenkel entsprechend dünn ausgebildet werden.

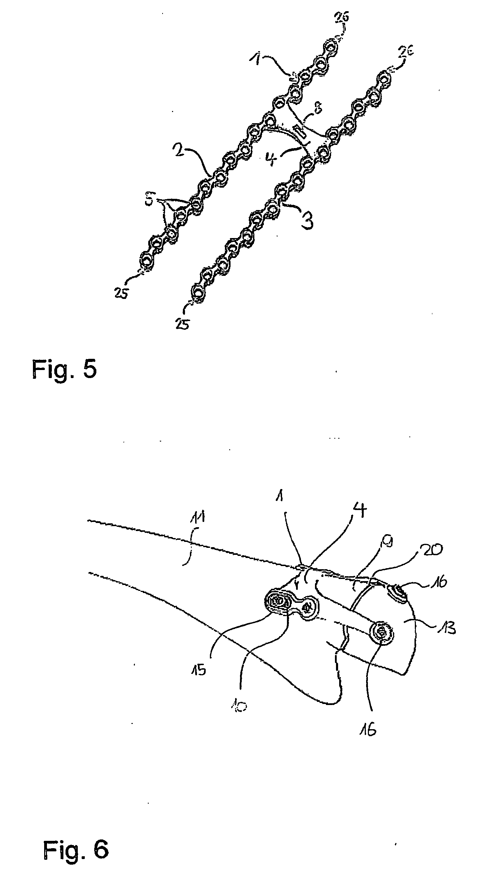

Figur 5 zeigt eine weitere alternative Ausführungsform der Osteosyntheseplatte 1. Dabei sind die Schenkel 2, 3 derart ausgebildet, dass die Öffnungen 5 nicht auf einer geraden Linie liegen, sondern jeweils gegeneinander seitlich versetzt, so dass die Form der Schenkel 2, 3 einer Schlangenlinie ähnelt. Die Breite der Schenkel 2, 3 ist zwischen den Öffnungen 5 kleiner als im Bereich der Öffnungen 5. Die erleichtert das Anpassen der Schenkel 2, 3 an die anatomischen Gegebenheiten des Knochens.

Figur 6 zeigt die Osteosyntheseplatte 1 gemäss Fig. 1 zur Versorgung einer Osteotomie am Olecranon. Die Figur zeigt der Einfachheit halber nur die Ulna 11 und keine weiteren Knochen des Ellbogengelenks. Diese Ausführungsform der Osteosyntheseplatte eignet sich besonders zur Versorgung von einfachen Osteotomien oder von nicht zertrümmerten Frakturen, bei denen nicht mehr als 2 Fragmente zu fixieren sind. Ein Bruch 20 im Olecranon wird mit der Platte 1 versorgt. Bevorzugt wird die Osteosyntheseplatte zunächst durch Biegen an die anatomischen Gegebenheiten angepasst. Wie in der Figur 3 gezeigt, werden die Schenkel dabei derart angebogen, dass deren epiphysären Enden 25 auf dem proximalen Fragment 13 des Olecranons zu liegen kommen. Die Osteosyntheseplatte 1 wird bevorzugt zuerst am proximalen/epiphysären Fragment mit zwei Knochenschrauben 16 fixiert. Danach kann eine Knochenschraube 15 im Langloch 10 platziert und leicht angezogen werden, so dass das Langloch 10 noch zu gleiten vermag. Mittels einer Zange lässt sich nun die Osteosyntheseplatte in distale/diaphysäre Richtung ziehen um eine Kompressionskraft auf die Fraktur- oder Osteotomiestelle auszuüben. Durch Festziehen der Schraube 15 im Langloch 10 und/oder durch Einsetzen weiterer Schrauben in die Öffnungen, die auf dem distalen/diaphysären Fragment liegen, lässt sich die Osteosyntheseplatte dann unter Beibehaltung der gewünschten Kompressionskraft fixieren.

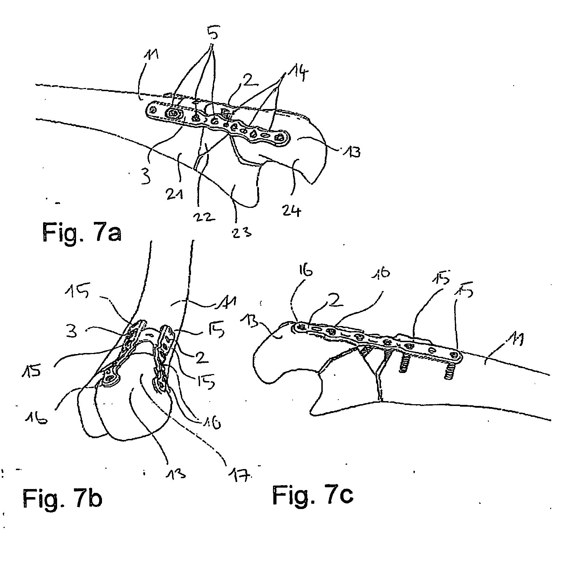

In Figuren 7a bis 7c ist eine weitere Ausführungsform der Osteosyntheseplatte 1 in verschiedenen Ansichten abgebildet, die derart ausgebildet ist, dass sie zur Versorgung einer zertrümmerten Fraktur mit mehreren Fragmenten 21, 22, 23, 24 am Olecranon anpassbar ist. Die Schenkel 2, 3 sind derart ausgebildet, dass sie sich beidseitig an die Ulna 11 und die epiphysären Enden sich leicht auf die proximale Fläche 13 des Olecranons durch Biegen anlegen lassen. Da bei dieser Ausführungsform die Osteosyntheseplatte nicht nur zur Aufnahme von Zugkräften dient, sondern mehrere Knochenfragmente möglichst stabil fixieren soll, ist sie dicker ausgebildet als die Platte in Figuren 1 und 3. Damit die Schenkel dennoch genügend gebogen werden können, weisen diese zwischen den Öffnungen 5 Einbuchtungen 14 auf. Für die Fixierung der multiplen Fragmente weisen die beiden Schenkel mehrere Öffnungen 5 auf. Mittels Knochenschrauben 15, 16 wird die Platte 1 an der Ulna 11 befestigt.

Wie die Figuren 6 und 7a bis 7c zeigen, bleibt das Olecranon dorsal im epiphysären Abschnitt 13 von der Osteosyntheseplatte 1 unbedeckt. Die Osteosyntheseplatte 1 stört daher den Patienten beispielsweise bei Auflage des Unterarms auf einen Tisch nicht und stört auch Weichgewebe in diesem Bereich nicht.

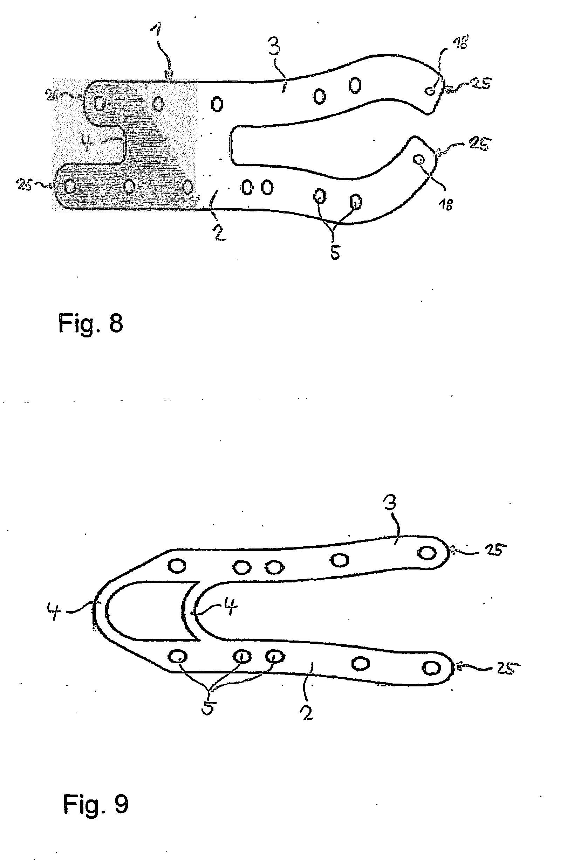

In Figur 8 ist eine weitere Ausführungsform der Osteosyntheseplatte 1 zu sehen. Die beiden Schenkel 2, 3 sind dabei in der Plattenebene leicht gekrümmt. Steg 4 erstreckt sich über eine grössere Fläche als die in Figur 1 gezeigte Ausführungsform. Öffnungen 5 sind asymmetrisch zueinander angeordnet, um die Wahrscheinlichkeit eines Aufeinandertreffens der Fixationselemente im Knochen zu minimieren. Die Form der vorgebogenen Schenkel 2, 3 eignet sich besonders, um die epiphysären Enden 25 der Schenkel 2, 3 über die äussere Krümmung der Epiphyse des Knochens zu biegen. Dabei bleibt der Bereich, bei dem Sehnen und Bänder in den Knochen einstrahlen, grösstenteils von der Platte unbeeinträchtigt. Über die beiden Öffnungen 18 am epiphysären Ende 25 der Schenkel 2, 3 lassen sich Fixationselemente in Richtung von epiphysärem Ende 25 zum diaphysären Ende 26 in den Knochen einbringen.

Figur 9 zeigt eine weitere Variante der Osteosynhteseplatte 1. Dabei sind die Schenkel 2, 3 durch zwei Stege 4 miteinander verbunden. Ein Steg verbindet dabei das diaphysäre Ende 26 der beiden Schenkel 2, 3 bogenförmig miteinander, während der zweite Steg die beiden Schenkel 2, 3 etwas weiter Richtung epiphysärem Ende 25 miteinander verbindet. Auch bei dieser Ausführungsform bleibt der Bereich, bei dem Sehnen und Bänder in den Knochen einstrahlen, grösstenteils von der Platte unbeeinträchtigt. Die zwei Stege 4 sind dabei so angeordnet, dass mindestens 60% der Länge L2 des kürzeren Schenkels 3 keine Verbindung zum längeren Schenkel 2 aufweist. Dadurch lässt sich die Platte von einer Seite her über die Frakturstelle auf den Knochen schieben. Dies wäre bei einer Platte, bei der die Stege z.B. an beiden Enden verbunden sind oder die Stege derart angeordnet sind, dass die Schenkel auf beiden Seiten nur über eine kurze Strecke nicht miteinander verbunden sind, nicht möglich.

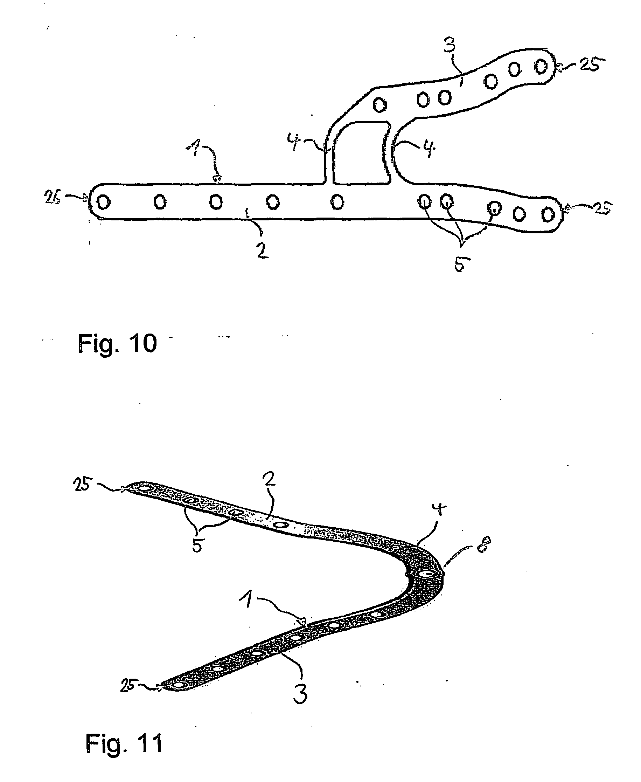

In Figur 10 ist eine Variante der Osteosyntheseplatte 1 aus Figur 9 zu sehen. Dabei erstreckt sich der längere Schenkel 2 wesentlich weiter als der kürzere Schenkel 3. Über den Schenkel 2 lassen sich somit auftretende Kräfte über eine grössere Fläche verteilen. Der Einsatz einer solchen Platte ist vor allem dort sinnvoll, wo auf einer Seite des Knochens wesentlich höhere Druckkräfte zu erwarten sind. Eine solche Platte kann auch eingesetzt werden, wenn in Richtung Diaphyse noch weitere Fragmente zu fixieren sind wie beispielsweise bei kombinierten Unterarm-Schaft-Frakturen. Schenkel 2 kann auch so ausgebildet werden, dass sich dessen Länge intraoperativ verkürzen lässt, z.B. mittels einer Schneidezange, um an die anatomischen Gegebenheiten des Knochens optimal angepasst zu werden.

Figur 11 zeigt eine Ausführungsform der Osteosyntheseplatte 1. Dabei sind die beiden Schenkel 2, 3 über einen einzelnen Steg 4 verbunden. Im Steg 4 ist mittig eine Aussparung 8 angeordnet, die bei dieser Ausführung auch zum Durchführen eines Fixationselements dienen kann. Der Steg 4 wird dabei über die äussere Kante der Epiphyse gebogen und die Schenkel 2, 3 an die Seiten des Knochens angelegt. Die Öffnungen 5 sind hier ebenfalls asymmetrische angeordnet. Eine solche Platte hat den Vorteil, dass die Fixierung nur über die Seiten des Knochens und dessen Epiphyse möglich ist. Dabei bleiben die Bereiche des Knochens, wo Sehnen oder Bänder einstrahlen, von der Platte grösstenteils unbeeinträchtigt.

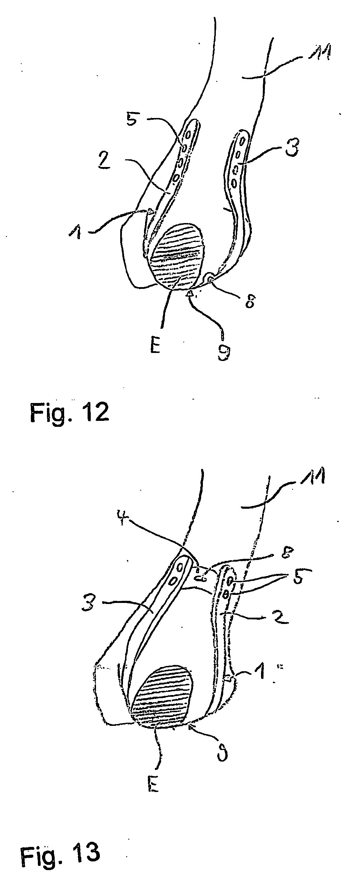

Figur 12 zeigt ein Anwendungsbeispiel der Osteosyntheseplatte 1 aus Figur 11 am Olecranon 9. Dabei wird der Steg 4 über die äussere Kante der Epiphyse, hier das Olecranon 9, gebogen. Die beiden Schenkel 2, 3 werden dabei beidseitig an die Seite der Ulna 11 angelegt. Durch die Aussparung 8 des Stegs 4 kann bei dieser Ausführungsform ein Fixationselement in den Knochen eingeführt werden, z.B. um die Osteosyntheseplatte 1 auf der Epiphyse zu verankern. Es können jedoch auch Öffnungen 5 derart angebracht sein, dass eine Verankerung der Platte 1 im Bereich der Epiphyse des Knochens durch diese Öffnungen erfolgen kann. Der Einstrahlbereich E von Bändern und Sehnen bleibt dabei von der Osteosyntheseplatte 1 gänzlich unbedeckt.

In Figur 13 ist ein Anwendungsbeispiel einer Osteosyntheseplatte 1 zu sehen, dessen Schenkel 2, 3 um die äussere Krümmung der Epiphyse, hier beispielhaft dem Olecranon 9, gebogen sind. Der Steg 4 ist derart ausgestaltet, dass er über die Zugseite des Knochens, hier die dorsale Kante der Ulna 11, gebogen werden kann. Die beiden Schenkel 2, 3 sind dabei derart angeordnet, dass sie an die seitlichen anatomischen Gegebenheiten des Knochens und der äusseren Krümmung der Epiphyse angepasst werden können. Dabei bleibt der Einstrahlbereich der Sehnen und Bänder sowie die dorsale Kante, die nur durch eine dünne Hautschicht bedeckt ist, von der Platte gänzlich unbedeckt oder nur unwesentlich durch den Steg 4 bedeckt.

## Patentansprüche

1. Osteosyntheseplatte (1) mit mindestens zwei zueinander im Abstand (D) beabstandeten und durch zwei Stege (4) miteinander verbundenen Schenkeln (2, 3), wobei die Schenkel (2, 3) je eine Länge (L1, L2) aufweisen und sich in Längsrichtung von einem epiphysären zu einem diaphysären Ende der Platte (1) erstrecken und über je mindestens eine Öffnung (5) zur Aufnahme von Fixationselementen (15, 16) verfügen, so dass Grösse und Form der Schenkel (2, 3) und der Stege (4) derart ausgebildet sind, dass die Schenkel (2, 3) beidseitig lateral an einen Knochen, insbesondere im Bereich eines Gelenks, anlegbar sind, bevorzugt am Olecranon (9) und dass die zwei Stege (4) und die Schenkel (2, 3) derart beschaffen sind, dass die zwei Stege (4) an die Krümmung des Knochens und die Schenkel (2, 3) an die seitlichen anatomischen Gegebenheiten des Knochens durch Biegen anpassbar sind **dadurch gekennzeichnet, dass** sich die zwei Stege in einem Kreuzungsbereich kreuzen, wobei der Kreuzungsbereich derart angeordnet ist, dass der Abstand zwischen dem Kreuzungsbereich (12) und dem epiphysären Ende (25) der Osteosyntheseplatte (1) mindestens 60% der Länge (L2) des kürzeren Schenkels (3) beträgt, wobei die mindestens zwei Schenkel (2, 3) und die zwei Stege (4) derart angeordnet sind, dass der Einstrahlbereich (F) von Sehnen und/oder Bändern nicht durch die Osteosyntheseplatte (1) abgedeckt wird.

2. Osteosyntheseplatte nach Anspruch 1, **dadurch gekennzeichnet, dass** die mindestens zwei Schenkel (2, 3) und die zwei Stege (4) derart angeordnet sind, dass auf der Zugseite des Knochens von der Kante der Epiphyse her ein Bereich von minimal 20mm, bevorzugt minimal 30mm in Richtung Diaphyse des Knochens von der Platte (1) nicht abgedeckt wird.

3. Osteosyntheseplatte nach einem der Ansprüche 1 oder 2, **dadurch gekennzeichnet, dass** mindestens einer der Schenkel (2, 3), bevorzugt alle Schenkel, derart beschaffen sind, dass sie sich an die äussere Krümmung der Epiphyse durch Biegen anpassen lassen, insbesondere so, dass ein in einer im epiphysären Ende (25) der Schenkel (2, 3) angeordnete Öffnung (18) eingesetztes Fixationselement etwa in Richtung von epiphysär zu diaphysär in den Knochen festlegbar ist.

4. Osteosyntheseplatte nach einem der Ansprüche 1 bis 3, **dadurch gekennzeichnet, dass** die Osteosyntheseplatte (1) im Bereich der wei Stege (4) weniger dick ist als im Bereich der Öffnungen (5), vorzugsweise etwa höchstens halb so dick.

5. Osteosyntheseplatte nach einem der Ansprüche 1 bis 4, **dadurch gekennzeichnet, dass** die Osteosyntheseplatte (1) im Bereich der zwei Stege (4) zwischen 0.1 und 0.7 mm, bevorzugt zwischen 0.2 mm und 0.6 mm dick ist und die Schenkel (2, 3) zumindest im Bereich der Öffnungen (5) zwischen 1.0 und 4.0 mm, bevorzugt zwischen 1.5 mm und 2.5 mm dick sind.

6. Osteosyntheseplatte nach einem der Ansprüche 1 bis 5, **dadurch gekennzeichnet, dass** die mindestens zwei Schenkel (2, 3) unterschiedliche Längen (L1, L2) aufweisen.

7. Osteosyntheseplatte nach einem der vorangehenden Ansprüche, **dadurch gekennzeichnet, dass** die zwei Stege (4) mindestens eine Aussparung (8) aufweisen

8. Osteosyntheseplatte nach einem der Ansprüche 1 bis 7, **dadurch gekennzeichnet, dass** der Abstand (D) der Schenkel zwischen 20 mm und 40 mm, bevorzugt zwischen 25 mm und 35 mm beträgt.

9. Osteosyntheseplatte nach einem der Ansprüche 1 bis 8, **dadurch gekennzeichnet, dass** die Öffnungen (5) in den mindestens zwei Schenkel (2, 3) zueinander asymmetrisch angeordnet sind.

10. Osteosyntheseplatte nach einem der vorangehenden Ansprüche, **dadurch gekennzeichnet, dass** die Länge (L2) des kürzeren Schenkels (3) zwischen 30mm und 150mm, bevorzugt zwischen 30mm und 40mm beträgt.

## Claims

1. Osteosynthesis plate (1) with at least two legs (2, 3), which are spaced apart from each other by a distance (D) and are connected to each other by two webs (4), wherein the legs (2, 3) each have a length (L1, L2) and extend in the longitudinal direction from an epiphyseal to a diaphyseal end of the plate (1) and each have at least one opening (5) for receiving fixing elements (15, 16), the size and shape of the legs (2, 3) and of the webs (4) being configured such that the legs (2, 3) can be applied laterally onto both sides of a bone, particularly in the area of a joint, preferably the olecranon (9), and the two webs (4) and the legs (2, 3) being configured such that the two webs (4) are adaptable, by bending, to the curvature of the bone, and the legs (2, 3) are adaptable, by bending, to the lateral anatomical circumstances of the bone, **characterized in that** the two webs intersect in an intersection area, wherein the intersection area is arranged in such a way that the distance between the intersection area (12) and the epiphyseal end (25) of the osteosynthesis plate (1) is at least 60% of the length (L2) of the shorter leg (3), wherein the at least two legs (2, 3) and the two webs (4) are arranged in such a way that the area (F) where tendons and/or ligaments radiate in is not covered by the osteosynthesis plate (1).

2. Osteosynthesis plate according to Claim 1, **characterized in that** the at least two legs (2, 3) and the two webs (4) are arranged in such a way that, from the edge of the epiphysis, an area of at least 20 mm, preferably of at least 30 mm, in the direction of the diaphysis of the bone is not covered by the plate (1) on the tension side of the bone.

3. Osteosynthesis plate according to either of Claims 1 and 2, **characterized in that** at least one of the legs (2, 3) and preferably all of the legs are configured in such a way that they can be adapted, by bending, to the outer curvature of the epiphysis, particularly in such a way that a fixing element, inserted into an opening (18) arranged in the epiphyseal end (25) of the legs (2, 3), can be secured in the bone substantially in the direction from the epiphysis to the diaphysis.

4. Osteosynthesis plate according to one of Claims 1 to 3, **characterized in that** the osteosynthesis plate (1) is less thick in the area of the two webs (4) than in the area of the openings (5), preferably approximately at most half as thick.

5. Osteosynthesis plate according to one of Claims 1 to 4, **characterized in that** the osteosynthesis plate (1) has a thickness, in the area of the two webs (4), of between 0.1 and 0.7 mm, preferably of between 0.2 mm and 0. 6 mm, and the legs (2, 3) have a thickness, at least in the area of the openings (5), of between 1.0 and 4.0 mm, preferably of between 1.5 mm and 2.5 mm.

6. Osteosynthesis plate according to one of Claims 1 to 5, **characterized in that** the at least two legs (2, 3) have different lengths (L1, L2).

7. Osteosynthesis plate according to one of the preceding claims, **characterized in that** the two webs (4) have at least one cutout (8).

8. Osteosynthesis plate according to one of Claims 1 to 7, **characterized in that** the distance (D) between the legs is between 20 mm and 40 mm, preferably between 25 mm and 35 mm.

9. Osteosynthesis plate according to one of Claims 1 to 8, **characterized in that** the openings (5) in the at least two legs (2, 3) are arranged asymmetrically with respect to one another.

10. Osteosynthesis plate according to one of the preceding claims, **characterized in that** the length (L2) of the shorter leg (3) is between 30 mm and 150 mm, preferably between 30 mm and 40 mm.

## Revendications

1. Plaque d'ostéosynthèse (1) comprenant au moins deux branches (2, 3) espacées l'une de l'autre d'une distance (D) et connectées l'une à l'autre par deux nervures (4), les branches (2, 3) présentant chacune une longueur (L1, L2) et s'étendant dans la direction longitudinale d'une extrémité épiphysaire à une extrémité diaphysaire de la plaque (1) et disposant chacune d'au moins une ouverture (5) pour recevoir des éléments de fixation (15, 16), de sorte que la taille et la forme des branches (2, 3) et des nervures (4) soient réalisées de telle sorte que les branches (2, 3) puissent être appliquées de chaque côté latéralement contre un os, en particulier dans la région d'une articulation, de préférence contre l'olécrâne (9), et que les deux nervures (4) et les branches (2, 3) soient réalisées de telle sorte que les deux nervures (4) puissent être adaptées à la courbure de l'os et que les branches (2, 3) puissent être adaptées par flexion aux données anatomiques latérales de l'os, **caractérisée en ce que** les deux nervures se croisent dans une région d'intersection, la région d'intersection étant disposée de telle sorte que la distance entre la région d'intersection (12) et l'extrémité épiphysaire (25) de la plaque d'ostéosynthèse (1) représente au moins 60 % de la longueur (L2) de la branche la plus courte (3), les au moins deux branches (2, 3) et les deux nervures (4) étant disposées de telle sorte que la région d'irradiation (F) des ligaments et/ou des tendons ne soit pas recouverte par la plaque d'ostéosynthèse (1).

2. Plaque d'ostéosynthèse selon la revendication 1, **caractérisée en ce que** les au moins deux branches (2, 3) et les deux nervures (4) sont disposées de telle sorte que du côté de traction de l'os depuis l'arête de l'épiphyse une région d'au moins 20 mm, de préférence d'au moins 30 mm ne soit pas recouverte par la plaque (1) dans la direction de diaphyse de l'os.

3. Plaque d'ostéosynthèse selon l'une quelconque des revendications 1 ou 2, **caractérisée en ce qu'**au moins l'une des branches (2, 3), de préférence toutes les branches, sont réalisées de telle sorte qu'elles puissent s'adapter par flexion à la courbure extérieure de l'épiphyse, notamment de telle sorte qu'un élément de fixation inséré dans une ouverture (18) disposée dans l'extrémité épiphysaire (25) des branches (2, 3) puisse être fixé approximativement dans la direction épiphysaire à diaphysaire dans l'os.

4. Plaque d'ostéosynthèse selon l'une quelconque des revendications 1 à 3, **caractérisée en ce que** la plaque d'ostéosynthèse (1) est moins épaisse dans la région des deux nervures (4) que dans la région des ouvertures (5), de préférence vaut approximativement au maximum la moitié de son épaisseur.

5. Plaque d'ostéosynthèse selon l'une quelconque des revendications 1 à 4, **caractérisée en ce que** la plaque d'ostéosynthèse (1), dans la région des deux nervures (4), a une épaisseur comprise entre 0,1 et 0,7 mm, de préférence entre 0,2 mm et 0,6 mm, et les branches (2, 3), au moins dans la région des ouvertures (5), ont une épaisseur comprise entre 1,0 et 4,0 mm, de préférence entre 1,5 mm et 2,5 mm.

6. Plaque d'ostéosynthèse selon l'une quelconque des revendications 1 à 5, **caractérisée en ce que** les au moins deux branches (2, 3) présentent des longueurs différentes (L1, L2).

7. Plaque d'ostéosynthèse selon l'une quelconque des revendications précédentes, **caractérisée en ce que** les deux nervures (4) présentent au moins un évidement (8).

8. Plaque d'ostéosynthèse selon l'une quelconque des revendications 1 à 7, **caractérisée en ce que** la distance (D) entre les branches est comprise entre 20 mm et 40 mm, de préférence entre 25 mm et 35 mm.

9. Plaque d'ostéosynthèse selon l'une quelconque des revendications 1 à 8, **caractérisée en ce que** les ouvertures (5) dans les au moins deux branches (2, 3) sont disposées de manière asymétrique les unes par rapport aux autres.

10. Plaque d'ostéosynthèse selon l'une quelconque des revendications précédentes, **caractérisée en ce que** la longueur (L2) de la branche la plus courte (3) est comprise entre 30 mm et 150 mm, de préférence entre 30 mm et 40 mm.
